# EUROPÄISCHE PATENTANMELDUNG

(11) **EP 0 794 171 A2**
(43) Veröffentlichungstag der Anmeldung: **10.09.1997**
(21) Anmeldenummer: 97102606.7
(22) Anmeldetag: 19.02.1997
(51) Int. Cl.: C07C 279/22, A61K 31/155

(54) **Ortho-substituierte Benzoylguanidine, Verfahren zu ihrer Herstellung, ihre Verwendung als Medikament oder Diagnostikum sowie sie enthaltendes Medikament**

(30) Priorität: 04.03.1996 DE 19608162
(71) Anmelder: HOECHST AKTIENGESELLSCHAFT, 65929 Frankfurt am Main (DE)
(72) Erfinder: Albus, Udo, Dr., 61197 Florstadt (DE); Brendel, Joachim, Dr., 61118 Bad Vilbel (DE); Kleemann, Heinz-Werner, Dr., 65474 Bischofsheim (DE); Lang, Hans Jochen, Dr., 65719 Hofheim (DE); Scholz, Wolfgang, Dr., 65760 Eschborn (DE); Schwark, Jan-Robert, Dr., 65929 Frankfurt (DE); Weichert, Andreas, Dr., 63329 Egelsbach (DE)

(57) **Zusammenfassung**

Ortho-substituierte Benzoylguanidine der Formel I 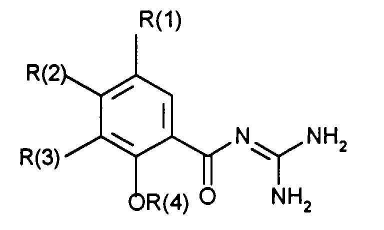 worin R(1) bis R(4) die in den Ansprüchen angegebenen Bedeutungen haben, sind als antiarrhythmische Arzneimittel mit cardioprotektiver Komponente zur Infarktprophylaxe und der Infarktbehandlung sowie zur Behandlung der angina pectoris geeignet.
Sie inhibieren auch präventiv die pathophysiologischen Vorgänge beim Entstehen ischämisch induzierter Schäden, insbesondere bei der Auslösung ischämisch induzierter Herzarrhythmien.

## Beschreibung

Die Erfindung betrifft Benzoylguanidine der Formel I 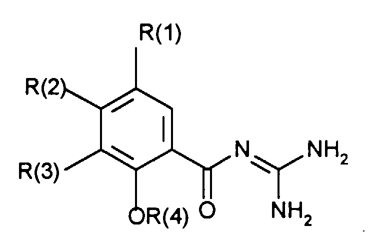 worin bedeuten:
- R(1): H, F, Cl, Br, I, CN, NO₂, Alkyl mit 1, 2, 3, 4, 5, 6, 7 oder 8 C-Atomen, Alkoxy mit 1, 2, 3, 4, 5, 6,7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
- X: Sauerstoff, S, NR(5),
- a: Null oder 1;
- b: Null, 1 oder 2;
- c: Null, 1, 2 oder 3;
- R (5): H, Alkyl mit 1, 2, 3 oder 4 C-Atomen oder -C_{d}H_{2d}R(6);
- d: Null, 1, 2, 3 oder 4;
- R(6): Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
- R(7) und R(8): unabhängig H oder Alkyl mit 1, 2, 3 oder 4 C-Atomen;
oder
- R(1): -SR(10), -OR(10) oder -CR(10)R(11)R(12);
- R(10): -C_{f}H₂f-Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
- f: Null, 1 oder 2;
- R(11) und R(12): unabhängig voneinander wie R(10) definiert oder Wasserstoff oder Alkyl mit 1, 2, 3 oder 4 C-Atomen;
oder
- R(1): Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
- R(1): -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]ₖ-(CO)-[CR(22)R(23)]ₗ-R(24),
- k: Null, 1, 2, 3 oder 4;
- l: Null, 1, 2, 3 oder 4;
- R(13) und R(14): gleich oder verschieden -(CH₂)g-(CHOH)ₕ-(CH2)ᵢ-(CHOH)ⱼ-R(17) oder -(CH₂)_{g}-O-(CH₂-CH₂O)ₕ-R(24);
- R(17): Wasserstoff oder Methyl,
- g, h und i: gleich oder verschieden Null, 1, 2, 3 oder 4;
- j: 1, 2, 3 oder 4;
- R(15) und R(16): gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
- R(18): Phenyl,
das unsubstituiert oder substituiert ist mit 1 - 3 Substituenten ausgewählt aus der Gruppe bestehend aus F, Cl, CF₃, Methyl, Methoxy und NR(25)R(26);
- R(25) und R(26):
- H: oder Alkyl mit 1, 2, 3 oder 4 C-Atomen;
oder
- R(18): Heteroaryl mit 1, 2, 3, 4, 5, 6, 7, 8 oder 9 Kohlenstoffatomen,
das unsubstituiert oder wie Phenyl substituiert ist;
oder
- R(18): Alkyl mit 1, 2, 3, 4,5 oder 6 C-Atomen,
das unsubstituiert oder mit 1 - 3 OH substituiert ist;
oder
- R (18): Cycloalkyl mit 3, 4, 5, 6, 7 oder 8 C-Atomen;
- R(19), R(20), R(21), R(22) und R(23): gleich oder verschieden Wasserstoff oder Methyl;
- R(24): H, Alkyl mit 1, 2, 3, 4, 5 oder 6 C-Atomen, Cycloalkyl mit 3, 4, 5, 6, 7 oder 8 C-Atomen oder -CₘH₂m-R(18);
- m: 1, 2, 3 oder 4;
- R(2) und R(3): wie R(1) definiert;
- R(4): Alkyl mit 1, 2, 3 oder 4 C-Atomen;
sowie deren pharmazeutisch verträgliche Salze.

Bevorzugt sind Verbindungen der Formel I, in denen bedeuten:
- R(1): H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
- X: Sauerstoff;
- a: Null oder 1;
- b: Null, 1 oder 2;
- c: Null, 1, 2 oder 3;
oder
- R(1): -SR(10) oder -OR(10);
- R(10): -C_{f}H_{2f}-Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
- f: Null oder 1;
oder
- R(1): Phenyl, Naphthyl, Biphenylyl oder Heteroaryl mit 1, 2, 3, 4, 5, 6, 7, 8 oder 9 C-Atomen, letzteres über ein C- oder N-Atom des Rings verknüpft,
die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
- R(1): -SR(13), -OR(13), -NHR(13), -NR(13)R(14),-C≡CR(18) oder -C[R(19)]=CHR(18);
- R(13) und R(14): gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
- R(17): Wasserstoff oder Methyl,
- g, h und i: gleich oder verschieden Null, 1 oder 2;
- j: 1 oder 2;
- R(18): Phenyl,
das unsubstituiert oder substituiert ist mit 1 - 3 Substituenten ausgewählt aus der Gruppe bestehend aus F, Cl, CF₃, Methyl, Methoxy und NR(25)R(26);
- R(25) und R(26):
- H: oder Alkyl mit 1, 2, 3 oder 4 C-Atomen;
oder
- R(18): Heteroaryl mit 1, 2, 3, 4, 5, 6, 7, 8 oder 9 Kohlenstoffatomen,
das unsubstituiert oder wie Phenyl substituiert ist;
oder
- R(18): Alkyl mit 1, 2, 3, 4, 5 oder 6 C-Atomen,
das unsubstituiert oder mit 1 - 3 OH substituiert ist;
oder
- R (18): Cycloalkyl mit 3, 4, 5, 6, 7 oder 8 C-Atomen;
- R(19): Wasserstoff oder Methyl;
- R(2) und R(3): wie R(1) definiert;
- R(4): Alkyl mit 1 oder 2 C-Atomen;
sowie deren pharmazeutisch verträgliche Salze.

Ganz besonders bevorzugt sind Verbindungen der Formel I, in denen bedeuten:
- R(1): H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder Xₐ-(CF₂)_{c}-CF₃;
- X: Sauerstoff;
- a: Null oder 1;
- c: Null oder 1;
oder
- R(1): -SR(10)oder-OR(10);
- R(10): Cycloalkyl mit 4, 5 oder 6 C-Atomen, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
- R(1): Chinolyl, Isochinolyl, Pyridyl,
die über ein C- oder N-Atom des Rings verknüpft sind;
und die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
- R(1): Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino,
oder
- R(1): -C≡CR(18);
- R(18): Phenyl oder Cycloalkyl mit fünf oder 6 C-Atomen;
- R(2) und R(3): wie R(1) definiert;
- R(4): Methyl;
sowie deren pharmazeutisch verträgliche Salze.

Besonders speziell bevorzugt sind Verbindungen der Formel I, in denen bedeuten
- R(1): H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder Xₐ-CF₃;
- X: Sauerstoff;
- a: Null oder 1;
- R(2) und R(3): wie R(1) definiert;
- R(4): Methyl;
sowie deren pharmazeutisch verträgliche Salze.

Unter Heteroaryl mit 1, 2, 3, 4, 5, 6,7, 8 oder 9 C-Atomen werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.
Als Heteroaryl mit 1, 2, 3, 4, 5, 6, 7, 8 oder 9 C-Atomen gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl; besonders Furanyl, Thienyl, Pyrrolyl, Imidazolyl, Pyrazolyl, Triazolyl, Thiazolyl, Pyridyl, Indolyl, Chinolyl und Isochinolyl.

Enthält einer der Substituenten R(1) bis R(4) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können geradkettig oder verzweigt sein.

Die Erfindung betrifft weiterhin ein Verfahren zur Herstellung der Verbindung I, dadurch gekennzeichnet, daß man eine Verbindung der Formel II 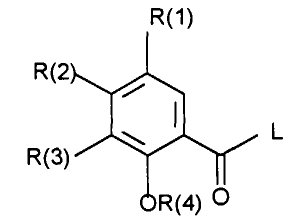 mit Guanidin umsetzt, worin R(1) bis R(4) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Eng. 1, 351 -367(1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorbo rat ("TOTU")[Weiss und Krommer, Chemiker Zeitung 98, 817 (1974)]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Losungsmittel. Dabei haben sich bei der Umsetzung der Benzoesäuremethylester (II, L = OMe) mit Guanidin Methanol, Isopropanol oder THF von 20^{°}C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Benzoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Den Verbindungen I ähnliche Verbindungen sind bekannt aus der Europäischen Offenlegungsschrift 640 588 A 1 (HOE 93/F 254). Jedoch enthalten die bekannten Verbindungen in 2-Stellung keinen Alkoxyrest, welcher die erfindungsgemäßen Verbindungen den bekannten gegenüber auszeichnet.

Ferner sind aus der Deutschen Offenlegungsschrift 44 21 495 desgleichen ähnliche Verbindungen bekannt, die aber stets einen Heteroaryloxy-Substituenten enthalten; die erfindungsgemäßen Verbindungen weisen dagegen diesen Heteroaryloxysubstituenten nicht auf.

Gegenüber den bekannten Verbindungen zeichnen sich die erfindungsgemäßen Verbindungen durch eine außerordentlich hohe Wirksamkeit in der Inhibition des Na⁺/H⁺-Austauschs aus.

Sie haben ebenso wie die bekannten Verbindungen keine unerwünschten und nachteiligen salidiuretischen, jedoch sehr gute antiarrhythmische Eigenschaften, wie sie beispielsweise zur Behandlung von Krankheitenwichtig sind, die bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na+/H+ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- RT: Raumtemperatur
- EE: Ethylacetat (EtOAc)
- Smp: Schmelzpunkt
- THF: Tetrahydrofuran
- eq: . Äquivalent

### Experimenteller Teil

### Allgemeine Vorschrift zur Herstellung von Benzoyl-guanidinen (I) Variante A: aus Benzoesäuren (II, L=OH)

1.0 eq. des Benzoesäurederivates der Formel II löst bzw. suspendiert man in wasserfreiem THF ( 5 ml/mmol ) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCl auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Sieden erhitzt (typische Reaktionszeit 2 bis 5 h). Das Lösungsmittel wird unter vermindertem Druck (Rotationsverdampfer) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1: 2,3-Dimethoxy-5-trifluormethylbenzoylguanidin-hydrochlorid: Farblose Kristalle,

Smp. 166^{o}C (Zersetzung)

### Syntheseweg:

a) 2-Chlor-3-iod-5-trifluormethyl-benzoesäuremethylester aus 2-Chlor-5-trifluormethyl-benzoesäuremethylester durch Reaktion mit 1 Äquivalent N-Iod-Succinimid in 5 Äquivalenten Trifluormethansulfonsäure bei RT für 24 h, farbloses
   Öl,
   (M+H)⁺:364
b) 2,3-Dimethoxy-5-trifluormethyl-benzoesäure aus 2-Chlor-3-iod-5-trifluormethylbenzoesäuremethylester (1 a) durch Reaktion mit 10 Äquivalenten 30%-Natriummethylat-Lösung in Gegenwart von 0.25 Äquivalenten Kupfer(II)chlorid in abs. DMF am Rückfluß innerhalb 1 h. Wäßrige Aufarbeitung und Extraktion mit EE liefert ein bräunliches Öl,
   (M+H)⁺: 251
c) 2,3-Dimethoxy-5-trifluormethyl-benzoylguanidin-hydrochlorid aus 1 b) nach Verfahren A, farblose Kristalle,
   Smp. 166^{o}C (Zersetzung).

### Beispiel 2: 2-Methoxy-3-iod-5-trifluormethyl-benzoylguanidin-hydrochlorid: Farblose Kristalle,

Smp. 150^{o}C (Zersetzung)

### Syntheseweg:

a) 2-Methoxy-3-iod-5-trifluormethyl-benzoesäure aus 1 a) durch Reaktion mit 1.1 Äquivalenten 30%-Natriummethylat-Lösung in Gegenwart von 0.3 Äquivalenten Kupfer(II)chlorid in abs. DMF bei RT innerhalb 2 h. Wäßrige Aufarbeitung und Extraktion mit EE liefert farblose Kristalle,
   Smp. 120-25^{°}C.
b) 2-Methoxy-3-iod-5-trifluormethyl-benzoylguanidin-hydrochlorid aus 2 a) nach Verfahren A, farblose Kristalle,
   Smp. 150^{o}C (Zersetzung)

### Beispiel 3: 2-Methoxy-3-cyclopentyl-5-trifluormethyl-benzoylguanidin-hydrochlorid: Farblose Kristalle,

Smp. 210^{o}C (Zersetzung)

### Syntheseweg:

a) 2-Chlor-3-cyclopentyl-5-trifluormethyl-benzoesäuremethylester aus 1 a) mittels cross-coupling mit 1.5 Äquivalenten Cyclopentylzinkchlorid (aus Cyclopentylmagnesiumchlorid durch Transmetallierung mit Zink(II)chlorid-Etherat in THF) durch Rühren bei RT in Gegenwart von katal. Palladium(II)[1,1'-bis-(diphenylphosphino)-ferrocen]chlorid und Kupfer(I)iodid, wäßrige Aufarbeitung, Extraktion mit Essigester und anschließende Säulenchromatographie an Kieselgel mit Essigesterln-Heptan (3:7),
   farbloses Öl, (M+H)⁺: 306
b) 2-Methoxy-3-cyclopentyl-5-trifluormethyl-benzoesäure durch Reaktion mit 10 Äquivalenten 30%-Natriummethylat-Lösung in Gegenwart von 0.25 Äquivalenten Kupfer(II)chlorid in abs. DMF am Rückfluß innerhalb 1 h. Wäßrige Aufarbeitung und Extraktion mit EE liefert ein farbloses Öl,
   (M+H)⁺: 289
c) 2-Methoxy-3-cyclopentyl-5-trifluormethyl-benzoylguanidin-hydrochlorid aus 3 b) nach Verfahren A, farblose Kristalle,
   Smp. 210^{o}C (Zersetzung)

### Beispiel 4: 2-Methoxy-5-trifluormethyl-benzoylguanidin-hydrochlorid Farblose Kristalle

Smp. 208^{°}C (Zersetzung)

### Syntheseweg:

a) 2-Methoxy-5-trifluormethyl-benzoesäure aus 2-Chlor-5-trifluormethylbenzoesäuremethylester durch Reaktion mit 10 Äquivalenten 30%-Natriummethylat-Lösung in Gegenwart von 0.25 Äquivalenten Kupfer(II)chlorid in abs. DMF bei 100^{°}C innerhalb 1.5 h. Wäßrige Aufarbeitung und Säulenchromatographie liefert farblose Kristalle,
   Smp. 108 - 10^{°}C
b) 2-Methoxy-5-trifluormethyl-benzoylguanidin-hydrochlorid aus 4 a) nach Verfahren A, farblose Kristalle,
   Smp. 208^{°}C (Zersetzung)

### Beispiel 5: 2-Methoxy-4-methyl-benzoylguanidin-hydrochlorid: Farblose Kristalle,

Smp. 213^{o}C.

### Syntheseweg:

a) 2-Methoxy-4-methyl-benzoesäure aus 2-Chlor-4-methyl-benzoesäuremethylester
   analog 3 b), farbloser Feststoff,
   Smp. 212^{o}C.
b) 2-Methoxy-4-methyl-benzoylguanidin-hydrochlorid
   aus 5 a) nach allg. Vorschrift.

### Beispiel 6: 3,5-Bis-tert.butyl-2-methoxy-benzoylguanidin-hydrochlorid: Farblose Kristalle,

Smp. 114^{o}C.

### Syntheseweg:

a) 3, 5-Bis-tert.butyl-2-methoxy-benzoesäuremethylester
   aus 3,5-Bis-tert.butyl-salicylsäuremethylester mittels Methyliodid in Gegenwart von Kaliumcarbonat in DMF,
   farbloses Öl, (M + H)⁺ = 278.
b) 3,5-Bis-tert.butyl-2-methoxy-benzoylguanidin-hydrochlorid
   aus 6 a) nach allg. Vorschrift.

### Beispiel 7: 2,4-Dimethoxy-5-trifluormethyl-benzoylguanidin-hydrochlorid: Farblose Kristalle,

Smp. 207^{o}C.

### Syntheseweg:

a) 2,4-Dimethoxy-5-brom-benzoesäuremethylester
   aus 5-Brom-2,4-hydroxy-benzoesäure mittels Methyliodid in DMF in Gegenwart von Kaliumcarbonat,
   farblose Kristalle,
   Smp. 115^{o}C.
b) 2,4-Dimethoxy-5-trifluormethyl-benzoesäuremethylester
   aus 7 a) durch Erhitzen auf 90°C mit Kaliumtrifluoroacetat in NMP in Gegenwart von Kupfer(I)iodid,
   farblose Kristalle,
   Smp. 125^{o}C.
c) 2,4-Dimethoxy-5-trifluormethvl-benzoylguanidin-hvdrochlorid:
   aus 7 a) nach allg. Vorschrift.

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na ⁺ /H ⁺ -Austausch zu aktivieren und so den Na ⁺-Influx in die Erythrocyten via Na ⁺ /H ⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37^{°}C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na ⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀(mol/l) |
| 1 | 0.053 x 10⁻⁶ |
| 2 | 0.017 x 10⁻⁶ |
| 5 | > 10⁻⁶ |
| 6 | 1 x 10⁻⁶ |
| 7 | 0.2 x 10⁻⁶ |

## Patentansprüche

1. Ortho-substituierte Benzoylguanidine der Formel I 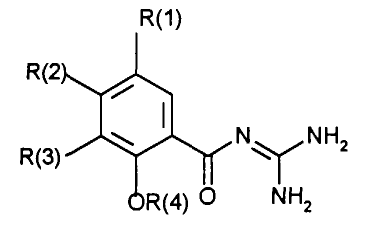 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
X Sauerstoff, S, NR(5),
a Null oder 1;
b Null, 1 oder 2;
c Null, 1, 2 oder 3;
R (5) H, Alkyl mit 1, 2, 3 oder 4 C-Atomen oder -C_{d}H_{2d}R(6);
d Null, 1, 2, 3 oder 4;
R(6) Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
R(7) und R(8)
unabhängig H oder Alkyl mit 1, 2, 3 oder 4 C-Atomen;
oder
R(1) -SR(10), -OR(10) oder -CR(10)R(11)R(12);
R(10) -C_{f}H₂f-Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert oder Wasserstoff oder Alkyl mit 1, 2, 3 oder 4 C-Atomen;
oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]ₖ-(CO)-[CR(22)R(23)]ₗ-R(24),
k Null, 1, 2, 3 oder 4;
l Null, 1, 2, 3 oder 4;
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
R(17) Wasserstoff oder Methyl,
g, h und i
gleich oder verschieden Null, 1, 2, 3 oder 4;
j 1, 2, 3 oder 4;
R(15) und R(16)
gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen,
das unsubstituiert oder wie Phenyl substituiert ist;
oder
R(18) Alkyl mit 1, 2, 3, 4, 5 oder 6 C-Atomen,
das unsubstituiert oder mit 1 - 3 OH substituiert ist;
oder
R (18)
Cycloalkyl mit 3, 4, 5, 6, 7 oder 8 C-Atomen;
R(19), R(20), R(21), R(22) und R(23)
gleich oder verschieden Wasserstoff oder Methyl;
R(24) H, Alkyl mit 1, 2, 3, 4, 5 oder 6 C-Atomen, Cycloalkyl mit 3, 4, 5, 6, 7 oder 8 C-Atomen oder -CₘH₂m-R(18);
m 1, 2, 3 oder 4;
R(2) und R(3)
wie R(1) definiert;
R(4) Alkyl mit 1, 2, 3 oder 4 C-Atomen;
sowie deren pharmazeutisch verträgliche Salze.

2. Verbindungen der Formel I nach Anspruch 1, dadurch gekennzeichnet, daß darin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
X Sauerstoff;
a Null oder 1;
b Null, 1 oder 2;
c Null, 1, 2 oder 3;
oder
R(1) -SR(10) oder -OR(10);
R(10) -C_{f}H_{2f}-Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null oder 1;
oder
R(1) Phenyl, Naphthyl, Biphenylyl oder Heteroaryl mit 1, 2, 3, 4, 5, 6, 7, 8 oder 9 C-Atomen, letzteres über ein C- oder N-Atom des Rings verknüpft,
die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14),-C≡CR(18) oder -C[R(19)]=CHR(18);
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
R(17) Wasserstoff oder Methyl,
g, h und i
gleich oder verschieden Null, 1 oder 2;
j 1 oder 2;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen,
das unsubstituiert oder wie Phenyl substituiert ist;
oder
R(18) Alkyl mit 1, 2, 3, 4, 5 oder 6 C-Atomen,
das unsubstituiert oder mit 1 - 3 OH substituiert ist;
oder
R (18)
Cycloalkyl mit 3, 4, 5, 6, 7 oder 8 C-Atomen;
R(19) Wasserstoff oder Methyl;
R(2) und R(3)
wie R(1) definiert;
R(4) Alkyl mit 1 oder 2 C-Atomen.

3. Verbindungen der Formel I nach Anspruch 1 oder 2, dadurch gekennzeichnet, daß darin bedeuten:
R(1) H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder Xₐ-(CF₂)_{c}-CF₃;
X Sauerstoff;
a Null oder 1;
c Null oder 1;
oder
R(1) -SR(10) oder-OR(10);
R(10) Cycloalkyl mit 4, 5 oder 6 C-Atomen, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(1) Chinolyl, Isochinolyl, Pyridyl,
die über ein C- oder N-Atom des Rings verknüpft sind;
und die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -C≡CR(18);
R(18)
Phenyl oder Cycloalkyl mit fünf oder 6 C-Atomen;
R(2) und R(3)
wie R(1) definiert;
R(4) Methyl.

4. Verbindungen der Formel I nach einem der Ansprüche 1 bis 3, dadurch gekennzeichnet, daß darin bedeuten:
R(1) H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder Xₐ-CF₃;
X Sauerstoff;
a Null oder 1;
R(2) und R(3)
wie R(1) definiert;
R(4) Methyl.

5. Verfahren zur Herstellung einer Verbindung I nach Anspruch 1, dadurch gekennzeichnet, daß man eine Verbindung der Formel II 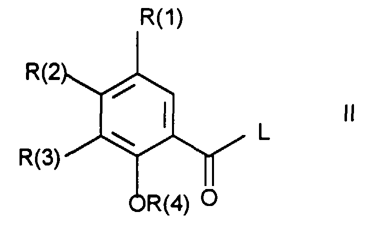 mit Guanidin umsetzt, worin R(1) bis R(4) die angegebene Bedeutung besitzen und L für eine leicht nucleophil substituierbare leaving group steht.

6. Verwendung einer Verbindung I nach Anspruch 1 zur Herstellung eines Medikaments zur Behandlung oder Prophylaxe von durch ischämische Zustände bewirkten Krankheiten.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie.

16. Arzneimittel, gekennzeichnet durch einen wirksamen Gehalt einer Verbindung der Formel I nach Ansprüchen 1 bis 4.
